# NEW EUROPEAN PATENT SPECIFICATION

(11) **EP 0 070 074 B2**
(45) Date of publication and mention of the opposition decision: **25.06.1997**
(45) Mention of the grant of the patent: 15.06.1988
(21) Application number: 82200868.6
(22) Date of filing: 12.07.1982
(51) Int. Cl.: C11D 1/83, C11D 1/66, C11D 1/72

(54) **Foaming surfactant compositions**
Schäumende, oberflächenaktive Verbindungen enthaltende Zusammensetzungen
Compositions moussantes contenant des agents tensio-actifs

(30) Priority: 13.07.1981 US 282976; 26.04.1982 US 371747
(43) Date of publication of application: 19.01.1983
(73) Proprietor: THE PROCTER & GAMBLE COMPANY, Cincinnati Ohio 45202 (US)
(72) Inventor: Llenado, Ramon A., West Chester, OH 45069 (US)
(74) Representative: Engisch, Gautier

(56) References cited:
- AT-B- 135 333
- DE-A- 2 110 030
- DE-A- 2 231 304
- DE-B- 953 422
- FR-A- 2 093 790
- GB-A- 976 088
- US-A- 3 547 828
- US-A- 3 653 095
- US-A- 3 721 633
- US-A- 3 839 318
- US-A- 3 925 224
- US-A- 4 154 706
- US-A- 4 240 921
- Rohm & Haas Technical Bulletin, Triton CG-110
- Rivista Italiana Ottobre 1974, pp. 567-572
- Nonionic Surfactants, 1967, pp. 683 and 693-697
- Journal of the American Chemical Society, 38, pp. 410-418, 1961
- Tensid Taschenbuch, 1981, pp. 302, 303, 310, 311
- Journal of the American Oil Chemist s Society, vol. 47, 1970, pp. 162-167
- Fettalkohole, pp. 126,127,98-101, 106, 107

## Description

### Technical Field

This invention relates to the use in dishwashing of compositions which provide controllable aqueous foams.

### Description of the prior art

Alkylpolyglycosides which are surfactants have been disclosed in U.S. patents 3,598,865; 3,721,633 and 3,772,269. These patents also disclose processes for making alkylpolyglycoside surfactants and built liquid detergent compositions containing these surfactants. In U.S. 3,721,633, anionic synthetic detergents can be present, in amounts of from 0 to 1 part by weight of anionic detergent per part of alkyl glycoside. U.S. patent 3,219,656 discloses alkylmonoglucosides and suggests their utility as foam stabilisers for other surfactants. Various polyglycosides surfactant structures and processes for making them are disclosed in U.S. patents 2,974,134; 3,640,998; 3,839,318; 3,314,936; 3,346,558, 4,011,389; 4,223,129.

Foaming compositions containing an alkylpolysaccharide surfactant, and anionic cosurfactants and possibly an auxiliary foam booster, have been disclosed in copending published EP-A-0070075 and EP-A-0070076.

Light duty detergent compositions containing an alkylpolysaccharide, alkylbenzene sulfonate and alkylpolyethoxylate sulfate cosurfactants are known from copending published EP-A-0070077.

All percentages, parts and ratios used herein are by weight unless otherwise specified.

### Summary of the invention

This invention relates to the discovery of dishwashing compositions which provide unusual foams. In particular the invention relates to use as a foaming dishwashing composition of a composition comprising
(1) an alkylpolyglucoside surfactant having the formula RO(R¹O)ₜZₓ wherein Z is a moiety derived from glucose, R is an alkyl group that contains from 12 to 18 carbon atoms preferably from about 12 to about 16 carbon atoms, most preferably from about 12 to about 14 carbon atoms; R¹ is ethylene, propylene and/or glyceryl, t is from 0 to 10, most preferably 0; wherein x is a number from 1.5 to 4, most preferably 1.6 to 2.7; and
(2) an anionic cosurfactant which is a sulfate, sulfonate and/or carboxylate or mixtures thereof neutralised with one or more cationic moieties (M) to complete the formula, preferably the anionic cosurfactant has the formula

   R⁹(SO₃)_{y}(COO)₂M_{q}

   wherein R⁹ is an alkyl, alkylphenyl, hydroxyalkylphenyl or hydroxyalkyl, or mixtures thereof, said alkyl groups containing from about 6 to about 30 carbon atoms, preferably about 10 to about 18 carbon atoms; y is a number from 0 to about 4, z is a number from 0 to about 4, y+z is at least 1, and M is a cationic moiety with q being selected to complete the formula, wherein the ratio (2) to (1) is from 1:10 to 10:1 and wherein the alkyl polyglucoside contains less than 50% short chain alkyl polyglucoside and less than 10% unreacted fatty alcohol, except that
   (1) the weight ratio of (2) to (1) is superior to 1:1 when the anionic surfactant is an alkalimetal alkylbenzenesulfonate and when in the general formula for the alkylpolyglucoside t=0
   (2) the weight ratio of (2) to (1) is at least 1:2 when the anionic cosurfactant is soap; and
   (3) when the anionic cosurfactant does not contain a sulfonate or carboxylate x must be from 1.5 to 3 and the alkylpolyglucoside surfactant must have a free fatty alcohol content of less than 2% by weight.

It has surprisingly been found that the cosurfactants interact with the alkylpolyglucoside surfactant of this invention to provide a relatively stable foam which is readily rinsed.

Certain compositions suitable for the defined use are described and claimed in EP-B-70075 and EP-B-70076, which have the same priority date.

### Description of the preferred embodiments

### The alkylpolyglucoside surfactant

The alkylpolyglucosides are those having an alkyl group containing from about 12 to about 18 carbon atoms, preferably from about 12 to about 16 carbon atoms, most preferably from 12 to 14 carbon atoms, and a polyglucoside hydrophilic group containing from about 1.5 to 4, most preferably from 1.6 to 2.7 glucoside units. The number x indicates the number of glucoside units in a particular alkylpolyglucoside surfactant. For a particular alkylpolyglucoside molecule x can only assume integral values. In any physical sample of alkylpolyglucoside surfactants there will be molecules having different x values. The physical sample can be characterised by the average value of x and this average value can assume non-integral values. In this specification the values of x are to be understood to be average values. The hydrophobic group (R) can be attached at the 2-, 3- or 4-positions rather than at the 1-position, (thus giving a glucosyl as opposed to a glucoside). However, attachment through the 1-position, i.e., glucosides, is preferred. In the preferred product the additional glucoside units are predominately attached to the previous glucoside unit's 2-position. Attachment through the 3-, 4- and 6-positions can also occur.

Optionally and less desirably there can be a polyalkyoxide chain joining the hydrophobic moiety (R) and the polyglucoside chain. The preferred alkoxide moiety is ethoxide.

Typical hydrophobic groups include alkyl groups, either saturated or unsaturated, branched or unbranched containing from about 12 to about 20, preferably from about 12 to about 16 carbon atoms. Preferably, the alkyl group is a straight chain saturated alkyl group.

Suitable alkyl polyglucosides are dodecyl, tetradecyl, hexadecyl, and octadecyl, di-, tri-, tetra-, penta- and hexaglucosides and mixtures thereof.

The alkylmonoglucosides are relatively less soluble in water than the higher alkylpolyglucosides. When used in admixture with alkylpolysaccharides, the alkylmonoglucosides are solubilised to some extent. The use of alkylmonoglucosides in admixture with alkylpolyglucosides is a preferred mode of carrying out the invention. Suitable mixtures include coconut alkyl, di-, tri-, tetra-, and pentaglucosides and tallow alkyl tetra-, penta-, and hexaglucosides.

The preferred alkyl polyglucosides have the formula

R²O(CₙH₂ₙO)ₜ(Z)ₓ

wherein Z is derived from glucose, R² is an alkyl group that contains from about 12 to about 18, preferably from 12 to 14 carbon atoms; n is 2 or 3, preferably 2, t is from 0 to about 10, preferably 0: and x is from 1.5 to 4, most preferably from 1.6 to 2.7. To prepare these compounds a long chain alcohol (R²OH) can be reacted with glucose, in the presence of an acid catalyst to form the desired glucoside. Alternatively the alkylpolyglucosides can be prepared by a two step procedure in which a short chain alcohol (C₁₋₆) is reacted with glucose or a polyglucoside (x=2 to 4) to yield a short chain alkyl glucoside (x=1 to 4) which can in turn be reacted with a longer chain alcohol (R²OH) to displace the short chain alcohol and obtain the desired alkylpolyglucoside. If this two step procedure is used, the short chain alkylglucoside content of the final alkylpolyglucoside material should be less than 50%, preferably less than 10%, more preferably less than 5%, most preferably 0% of the alkylpolyglucoside.

The amount of unreacted alcohol (the free fatty alcohol content) in the desired alkylpolyglucoside surfactant is preferably less than about 2%, more preferably less than about 0.5% by weight of the total of the alkylpolyglucoside plus unreacted alcohol. The amount of alkylmonoglucoside is about 20% to about 70%, preferably 30% to 60%, most preferably 30% to 50% by weight of the total of the alkylpolyglucoside. for some uses it is desirable to have the alkylmonoglucoside content less than about 10%.

### The anionic cosurfactants

Anionic cosurfactants can be selected from the group consisting of sulfates, sulfonates, carboxylates and mixtures thereof. The cosurfactants are neutralised with a cationic moiety or moieties selected from the group consisting of alkali metal, e.g., sodium or potassium, alkaline earth metal, e.g., calcium or magnesium, ammonium, substituted ammonium, including mono-, di-, or tri-, ethanolammonium cations. Mixtures of cations can be desirable. The anionic cosurfactants useful in the present invention all have detergent properties and are all water-soluble or dispersible in water.

### Alkylbenzene sulfonates

One of the preferred cosurfactants for use in this invention is an alkylbenzene sulfonate. The alkyl group can be either saturated or unsaturated, branched or straight chain and is optionally substituted with a hydroxy group. Middle phenyl positions are generally preferred for volume of foaming in light soil conditions. However, in heavier soil conditions, phenyl attachment at the 1- or 2-position is preferred.

The preferred alkylbenzene sulfonates contain a straight alkyl chain containing from about 9 to about 25 carbon atoms, preferably from about 10 to about 13 carbon atoms, and the cation is sodium, potassium, ammonium, mono-, di-, or triethanolammonium, calcium or magnesium and mixtures thereof. Magnesium is the preferred cationic moiety. These same cations are preferred for other anionic surfactants and ingredients. The magnesium alkylbenzene sulfonates where the phenyl group is attached near the middle of the alkyl chain are surprisingly better than the ones with the phenyl near the end of the chain when the polysaccharide chain averages greater than about 3 saccharide units Suitable alkylbenzene sulfonates include C₁₁ alkylbenzene sulfonates with low 2-phenyl content (i.e. majority of phenyl groups attached uses the middle of alkyl chain).

The alkylbenzene sulfonate cosurfactant is desirable in the foaming compositions of the invention since the foams produced therewith are exceptionally stable, have a large volume, rinse quickly, and do not have a "slippery" feel. These compositions are particulalty desirable for industrial and commercial processes as discussed hereinafter. The volume of foam produced using the alkylbenzene sulfonate cosurfactant is larger than for any other cosurfactant.

### Soap

Other preferred cosurfactants for use in this invention are carboxylates, e.g. fatty acid soaps and similar surfactants. The soaps can be saturated or unsaturated and can contain various substituents such as hydroxy groups and alpha-sulfonate groups. Preferably, the hydrophobic portion of the soap is a straight chain saturated or unsaturated hydrocarbons. The hydrophobic portion of the soap usually contains from about 6 to about 30 carbon atoms, preferably from about 10 to about 18 carbon atoms. The use of carboxylate cosurfactants is especially valuable since the alkylpolysaccharide surfactants are exceptional lime soap dispersers.

The cationic moiety (M) for carboxylate cosurfactants is selected from the group consisting of alkali metal, for examples sodium or potassium, alkaline earth metal, for example, calcium or magnesium, ammonium, or substituted ammonium, including mono-, di-, or triethanolammonium cations. Mixtures of cations can be desirable.

In addition to the preferred alkylbenzene sulfonate and soap cosurfactants many other surfactants which contain sulfonate or carboxylate groups can be used in the foaming compositions used in the invention. Generally the use of these latter cosurfactants produces less foam volume than does the use of the preferred cosurfactants. However, the alkylpolysaccharide surfactant stabilizes the foams which are produced and allows the foams to be rinsed more quickly.

Yet other cosurfactants are the alkyl (paraffin or olefin sulonates, preferably with a more central hydrophilic group, containing from about 6 to about 30 carbon atoms. Compositions containing these cosurfactants produce the least volume of foam, if that is desired. The hydrophobic group can contain up to about 10 hydroxy groups and/or ether linkages. Examples include C₁₄₋₁₅ paraffin sulfonates and C₁₄₋₁₆ olefin sulfonates.

Still another cosurfactant is a soap structure containing up to about 10 ether linkages in the chain and from about 1 to about 4 carbon atoms between ether linkages with from about 6 to about 30 carbon atoms in a terminal portion containing no ether linkages

The alkylpolyglucosides that contain an average of from 1.5 to 4 glucoside units, preferably from 1.6 to 2.7 glucoside units; less than about 50% short chain alkylpolyglucosides; less than about 10%, preferably less than about 2%, most preferably less than about 0.5% unreacted fatty alcohol, increase the sudsing ability of conventional sulfate detergent cosurfactants, especially alkyl sulfate and alkyl polyether sulfate cosurfactants having the formula:

R³O(CₙH₂ₙO)ₜSO₃M

wherein R³ is an alkyl or hydroxyalkyl group containing from about 8 to about 18 cabron atoms, n is 2 or 3, t can vary from 0 to about 30, and M is a cationic moiety as defined above, the cosurfactant being water soluble or dispersible.

A preferred foaming composion for use in the invention herein comprises: (1) an alkylpolysaccharide surfactant having the formula RO(R¹O)ₜ(Z)ₓ, wherein Z is a moiety derived from a reducing saccharide containing from 5 to 6 carbon atoms and wherein R is a hydrophobic group selected from the group consisting of alkyl, alkylphenyl and mixtures thereof in and which said alkylgroups contain from about 8 to about 18 carbon atoms, R¹ is ethylene, propylene and/or glyceryl, t is from 0 to about 30; and x is a number from about 1.5 to about 10, preferably 1.5 to 4, most preferably 1.6 to 2.7; and
(2) a mixture of cosurfactants neutralized with one or more cationic moieties consisting essentially of:
(a) from about 1% to about 95% preferably about 10% to about 50% of a water soluble alkylbenzene sulfonate cosurfactant in which the alkyl group contains from about 10 to about 13 carbon atoms, and (b) from about 5% to about 99%, preferably 50-90% of a cosurfactant selected from the group consisting of an alkyl glyceryl ether sulfonate in which the alkyl group contains from about 8 to about 18 carbon atoms, an alpha-olefin sulfonate in which the olefin group contains from about 10 to about 18 carbon atoms, an alkyl polyethoxylate carboxylate in which the alkyl group contains from about 10 to about 18 carbon atoms, and the polyelhoxylate chain contains from about 2 to about 6 ethoxylate groups, and mixtures thereof.

Such compositions have improved suds mileage as compared to compositions containing only the alkyl benzene sulfonate cosurfactant and the alkylpolysaccharide surfactant.

Another preferred embodiment of a foaming composition for use in the invention herein comprises:
(1) an alkylpolysaccharide surfactant having the formula RO(R¹O)ₜ(Z)ₓ wherein Z is a moiety derived from a reducing saccharide containing from 5 to 6 carbon atoms and wherein R is a hydrophobic group selected from the group consisting of alkyl, alkylphenyl and mixtures thereof in which said alkyl groups contain from about 8 to about 18 carbon atoms; R¹ is ethylene, propylene and or glyceryl; t is from 0 to about 30; and x is a number from about 1.5 to about 10;
(2) is anionic cosurfactant selected from the group consisting of sulfates, sulfonates, carboxylates and mixtures thereof neutralized with one or more cationic moieties M to complete the formula, the ratio of (2) to (1) being from about 1:10 to about 10:1, and
(3) from about 2% to about 10% of an auxiliary foam booster selected from the group consisting of:
   (a) amides having the formula 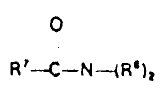 wherein R⁷ is an alkyl group containing from about 8 to about 18 carbon atoms, preferably about 12 to about 14 carbon atoms and each R⁸ is the same or different and is selected from the group consisting of hydrogen, C₁₋₃ alkyl, C₁₋₃ alkanol, and -(CH₂O₄-)₁₋₄H groups and mixtures thereof;
   (b) amine oxides having the formula: 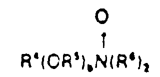 wherein R⁴ is an alkyl group containing from about 8 to about 18 carbon atoms, preferably from 12 to 14 carbon atoms, each R⁴ contains two or three carbon atoms, b is from 0 to about 30, each R⁶ is the same or different and is selected from the group consisting of C₁₋₃ alkyl, C₁₋₃ alkanol, and -(C₂H₄O)₁₋₆H groups and mixtures thereof; and
   (c) mixtures thereof.

Such compositions provide superior grease/oil removal and suds mileage.

Preferred anionic cosurfactants are alkylbenzene sulfonate, alpha-olefin sulfonate, alkylsulfates, alkylpolyethoxylate sulfates and paraffin sulfonates and mixtures thereof. The cationic moieties are selected from the group consisting of sodium, potassium, ammonium, monoethanolammonium, diethanolammonium, triethanolammonium, calcium, magnesium and mixtures thereof.

Preferred compositions for use in this embodiment of the invention comprise from 1% to about 95%. preferably 5% to about 50% of an alkypolyglucoside surfactant in which the alkyl group contains from 12 to 14 carbon atoms, x is from 1.5 to 4, more preferably 1.6 to 2.7; from 1% to about 95%, preferably from about 10% to about 50% of an anionic cosurfactant neutralized with one or more cationic moieties and which is a mixture of
(1) from 1% to about 95%, preferably from about 5% to about 50% of an alkyl benzene sulfonate in which the alkyl group contains from about 8 to about 13 carbon atoms or an alpha-olefin sulfonate in which the olefin group contains from about 10 to about 18 carbon atoms, or mixtures thereof; and
(2) from 1% to about 95%, preferably from about 5% to about 50% of an alkyl polyethoxylate sulfate in which the alkyl group contains from about 8 to about 18 carbon atoms, preferably from 12 to 14 carbon atoms and from about one to about six ethoxylate moieties and wherein from about 1% to about 100%, preferably from about 10% to about 80% of the cationic moieties are magnesium; and
wherein the auxiliary foam booster is an amide.

Another preferred foaming composition for use in the invention herein is an agglomerated light duty detergent granule composition comprising
(1) from about 5% to about 60%, preferably from 10% to about 20% of an alkylpolysaccharide surfactant having the formula RO(R¹O)ₜ(Z)ₓ, wherein Z is a moiety derived from a reducing saccharide moiety containing from 5 to 6 carbon atoms and wherein R is a hydrophobic group selected from the group consisting of alkyl, alkylphenyl and mixtures thereof in which said alkyl groups contain from about 8 to about 18 carbon atoms, preferably from 12 to 14 carbon atoms; R¹ is ethylene, propylene and/or glyceryl: t is from 0 to about 30; and x is a number from about 1.5 to about 10, preferably 1.5 to 4, most preferably 1.6 to 2.7;
(2) from about 5% to about 60% of an alkyl benzene sulfonate cosurfactant in which the alkyl group contains from about 10 to about 13 carbon atoms, said alkyl benzene sulfonate neutralized with one or more cationic moieties (M) to balance the formula
(3) from about 5% to about 60%, preferably from about 10% to about 20% of an alkylpolyethoxylate sulfate cosurfactant in which the alkyl group contains from about 10 to about 16 carbon atoms and in which there are from 1 to about 6 ethoxylate groups, said alkylpolyethoxylate sulfate neutralized with one or more cationic moieties M to complete the formula;
(4) from about 5% to about 80% of a water soluble inorganic salt selected from the group consisting of sodium and potassium sulfates, chlorides, carbonates, phosphates, and mixtures thereof.

### The processes

Mixtures of alkylbenzene sulfonate and/or the soap cosurfactant and the alkylpolysaccharide surfactant can be used at levels of from about 0.01% to about 95%, in ratios of cosurfactant to alkylpolysaccharide of from about 10:1 to about 1:10, in water with agitation to provide foams. These foams are relatively stable and, if not disturbed, can exist for several days. Furthermore, the foam has structural integrity and does not spread out. The foams prepared using mixtures of alkylbenzene sulfonate and the alkylpolysaccharide are unique in that they do not have a "slippery" feel. All of the foams rinse quickly.

Typical compositions for use as light duty liquid detergent compositions in washing dishes comprise from about 5% to about 50%, preferably from about 10% to about 40% of the mixture of surfactants disclosed hereinbefore. From about 1% to about 50% of a solvent selected from the group consisting of C₁₋₃ alkanols, C₁₋₃ alkanolamines, C₂₋₄ polyols, mixtures thereof, and the balance water. It is a special advantage of the compositions for use in this invention that they can be made in concentrated form (up to about 50% by wt, of the mixture of surfactants) with only very low levels of organic solvents and without the addition of expensive hydrotropic materials. Additional suds boosters or builders such as trialkyl amine oxides and fatty acid amides can also be used in amounts up to about 20%. Fatty alcohols should not be used.

### Additional ingredients

The processes of this invention can utilize other compatible ingredients, including other surfactants, in addition to the mixture of surfactants herein disclosed. In detergent compositions the compositions can contain any of the well known ingredients including minor amounts of other surfactants, detergency builders, soil suspending agents, brighteners, abrasives, dyes, hydrotropes, solvents, filters, clays, perfumes, etc. Suitable ingredients are disclosed in US. Patents 4,660,039-Wise, 4,157,978-Llenado, 4,056,481-Tate, 4,049,586-Collier, 4,035,257-Cherney, 4,019,998-Benson et al; 4,000,080-Bartolotta et al; and 3,983,078-Collins, incorporated herein by reference. Listings of suitable additional ingredients, including low levels of other surfactants can be found in U.S. Patents 4,089,945; 3,987,161; and 3,962,418, incorporated herein by reference.

Of special interest are ingredients which modify the feel of aqueous solutions containinq the foaming compositions of this invention. For examples raising the pH to above about 8.5 by alkaline materials or incorporating the tertiary alcohols of the European patent application No. 0 049 546, published on April 14, 1982. Such ingredients are desirable for some consumers since the solutions do not have the normal "soapy" feel associated with surfactant solutions.

The following nonlimiting examples illustrate the foaming compositions for use in the present invention.

### Example I

| Relative volume of suds comparison and consumer preference | | | |
|---|---|---|---|
| | A | B | C |
| | Generic commercial product U.S. crystal White® | Premium commercial product U.S. of the Palmolive® | Product of the invention |
| Formula | Weight % | Weight % | Weight % |
| Sodium C_{11.8} alkyl benzene sulfonate | 10.5 | 18.0 | 18.0 |
| C₁₂₋₁₃ alkylpolyglucoside₂₋₃ (>2% free fatty alcohol) | - | - | 12.0 |
| Sodium C₁₄₋₁₅ alkyl polyethoxylate, sulfate | 5.5 | 12.0 | - |
| Balance of formula inc. water | 84.0 | 70.0 | 70.0 |
| Sudsing Relative Volume of Suds (ml) 0.2% solutions | 110 | 125 | 220 |
| Consumer test Overall preference. % | 10 | 18 | 23 |
| Favorable sudsing comments. % | 74 | 85 | 80 |
| Favorable rinsing comments. % | 3 | 6 | 10 |

The foaming composition for use in the invention is superior to a representative generic product and at least equivalent to a representative premium commercial product and is preferred by consumers for rinsing reasons. The test involved 50 consumers washing soiled dishes in the test solutions. The consumers wore rubber gloves during the test. The differences are significant at the 95% confidence level for the invention over the generic product.

The relative volume of suds in ml. is determined by the following test procedure:
100 ml of the test solution at 46.1°C is placed in a 500 ml graduated cylinder: the solution is agitated by repeated inversion of the graduated cylinder until the amount of suds in the cylinder does not increase with further agitation. Suds height is measured directly on the cylinder scale making allowance for the height of liquid remaining in the cylinder. The test solution is made by adding the test product to water having a hardness of 0.12 grams per liter (Ca/Mg=3/1).

### Example II

| LAS suds boosting | | | | | | | |
|---|---|---|---|---|---|---|---|
| Wt. % of: | A | B | C | D | E | F | G |
| Sodium C_{11.8} alkylbenzene sulfonate | 0 | 20 | 40 | 50 | 60 | 80 | 100 |
| C₁₂₋₁₅ Alkylpoly₂₋₃ glucoside | 100 | 80 | 60 | 50 | 40 | 20 | 0 |
| Relative Volume of suds (ml) | 140 | 220 | 280 | 300 | 310 | 300 | 240 |

The suds (foam) were generated as described in Ex. 1 using 300 ppm of the surfactant mixtures in city water (0.15 grams per liter). The results clearly show the sudsing synergism for ratios greater than about 1 2. The foaming composition of the invention herein correspond to formulae E,F.

### Example III

| Soap suds boosting | | | | | | |
|---|---|---|---|---|---|---|
| Wt. % of: | A | B | C | D | E | F |
| Sodium oleate | 0 | 20 | 40 | 60 | 80 | 100 |
| C₁₂₋₁₅ alkylpoly₂₋₃ glucoside | 100 | 80 | 60 | 40 | 20 | 0 |
| Relative Volume of suds (ml.) | 160 | 270 | 280 | 300 | 310 | 260 |

The suds were generated as in Ex. 1 using 500 ppm, of the surfactant mixtures. This data clearly indicates the sudsing synergism for the foaming composition for use in the invention.

### Example IV

| Foaming With soap Effect of hardness on relative volume of suds | | | | |
|---|---|---|---|---|
| | Grains hardness | | | |
| | 0 | 2 | 4 | 6 |
| Sodium oleate | 225 | 10 | 0 | 0 |
| Sodium oleate plus C₁₂₋₁₅ alkylpoly₂₋₃ glucoside (3:2 ratio) | 360 | 100 | 55 | 10 |

The suds were generated as in Ex. 1 using 500 ppm, of the surfactant mixtures.

### Example V

| Alkyl polyglucoside (C₁₂₋₁₅ alkylpoly₂₋₃ glucoside) Suds boosting for the following representative cosurfactants (3 2 ratio. 500 ppm) | |
|---|---|
| | % increase in foaming |
| Sodium C_{11.8} allylbenzene sulfonate | 100-150 |
| Sodium oleate | 50-75 |
| 3-[N-coconutalkyl-N,N-dimethyl]- 2-hydroxy-1-sulfonate | 40-60 |
| Sodium C₁₄₋₁₅ olefin sulfonate | 20-40 |
| Sodium coconut alkyl sulfate | 10-30 |
| Sodium coconut alkyl polyethoxylate₃ sulfate | 0-20 |

The above data clearly demonstrate the criticality of utilizing a carboxylate or sulfonate anionic detergent cosurfactant for sudsing synergism with the alkyl polyglucoside surfactant.

### Example VI

| Alkylbenzene sulfonates (LAS) Homologs/phenyl-position (3 2 ratio; 500 ppm) | |
|---|---|
| | Relative volume of suds (ml) |
| Ex. Il's alkylpolyglucoside plus: | |
| Sodium C₁₁ LAS, high* 2-phenyl | 210 |
| Sodium C₁₁ LAS. low** 2-phenyl | 250 |
| Sodium C₁₂ LAS, high 2-phenyl | 225 |
| Sodium C₁₂ LAS, low 2-phenyl | 225 |
| Sodium C₁₄ LAS, high 2-phenyl | 210 |
| Sodium C₁₄ LAS, low 2-phenyl | 215 |
| As can be seen from the above, in general C₁₁, low 2-phenyl LAS is preferred for sudsing. | |

| | |
|---|---|
| *phenyl group attached over the end of the alkyl chain | |
| **phenyl group attached near the middle of the alkyl chain. | |

### Example VII

| Suds boosting of alkyl polyglucosides and effect of soil | | | |
|---|---|---|---|
| | Relative volume of suds (ml) | | |
| | Without soil | With soil* | |
| | | 0.5% | 1.0% |
| 0.2.% aqueous solution of a detergent composition formulated with: 15% sodium C_{11.8} alkylbenzene sulfonate (C_{11.8} LAS) | 120 | 50 | 25 |
| 15% C_{11.8} LAS+12% Ex. II's alkyl polyglucoside | 310 | 130 | 70 |
| 30% C_{11.8} LAS | 190 | 140 | 100 |

| | | | |
|---|---|---|---|
| *Test method of Ex. I modified by adding to the test solution the indicated amount of soil. % is wt.% of test solution. | | | |

The soil is a 44%/56% by weight mixture of Fluffo® and PREP® both of which products are available in the United States from The Procter & Gamble Company.

As can be seen from the above, the benefit for the invention is even more remarkable when soil is present.

### Example VIII

| Relative volume of suds (ml) | | |
|---|---|---|
| | No soil | 1% soil* present |
| Generic commercial product (Crystal While®)** | 110 | 30 |
| Premium commercial product B (Palmolive Liquid®) | 120 | 100 |
| Premium commercial product C (Joy®) | 125 | 120 |
| 12% C_{11.8} LAS/8% Ex. II's alkyl polyglucoside | 180 | 120 |
| 18% C_{11.8} LA/12% Ex. II's alkyl polyglucoside | 240 | 150 |
| 24% C_{11.8} LA/16% Ex. II's alkyl polyglucoside | 300 | 180 |

| | | |
|---|---|---|
| *Soil is added as described in Ex. VII. | | |
| **Crystal While® is available from Colgate-Palmolive Co. Palmolive Liquid ® is available from Colgate-Palmolive Co. Joy® is available from The Procter & Gamble Company. | | |

Suds generated as in Ex. I using a test solution containing 0.2% by wt, of the indicated commercial product or 0 2% of a product formulated with the surfactant mixtures shown.

As can be seen, the simple mixtures of surfactants representative of this invention can be formulated to be superior, or at least equal, to even the best light-duty dishwashing liquids.

### Example IX

| Wt. % of: | A | B | C | D | E | F |
|---|---|---|---|---|---|---|
| C_{11.8} LAS (Sodium) | 0 | 20 | 40 | 60 | 80 | 100 |
| Sucrose monolaurate | 100 | 80 | 60 | 40 | 20 | 0 |
| Relative Volume of suds (ml.) | 30 | 100 | 150 | 190 | 210 | 220 |

300 ppm of surfactant mixture used in test solution of Ex. I.

The above demonstrates that structures which are similar to the alkyl polyglucosides do not provide the benefits of this invention.

### Example X

| Sodium vs. magnesium alkybenzene sulfonate Relative volume of suds (ml.) | | | |
|---|---|---|---|
| | Without soil | With soil* | |
| | | 0.6% | 1.0% |
| 0.2% aqueous solution of a detergent composition with: 15% Ex., II's alkyl polyglucoside: 22% C_{11.8} alkylbenzene sulfonate with the benzene group attached primarily to the center of the alkyl chain, sodium neutralized | 450 | 150 | 75 |
| 15%, Ex. II's alkyl polyglucoside; 22% C_{11.8} alkylbenzene sulfonate with the benzene group anached primarily to the center of the alkyf chain, magnesium neutralized | 450 | 200 | 110 |
| Premium product (Joy®) | 350 | 120 | 75 |

| | | | |
|---|---|---|---|
| *Soil added to the test solution as in Ex. VII. | | | |

### Example XI

The optimum alkylpolyglucosides have an HLB* of from about 6 to about 27 and a critical micelle concentration (CMC)** of less than about 1000 ppm, preferably less than about 500. Short chain alkylpolyglucosides which the alkyl group contains less than about 8 carbon atoms have unacceptably high CMC's and those alkylpolyglucosides having more than about 4 glucoside units have unacceptably high HLB'S as is shown in the following table in which the alkyl group and the glucoside chain length were varied.

| * of Glucosides ** of Carbons | | G₀ | G₁ | G₂ | G₃ | G₄ | G₅ |
|---|---|---|---|---|---|---|---|
| C₄ | HLB | 5.1 | 12.4 | 17.9 | 23.3 | 28.8 | 34.2 |
| C₆ | HLB | 4.2 | 11.4 | 17.0 | 22.4 | 27.8 | 33.2 |
| C₈ | HLB | 3.2 | 10.5 | 16.0 | 21.4 | 26.9 | 32.3 |
| | CMC | | ∼7000 | | | | |
| C₁₀ | HLB | 2.2 | 9.6 | 15.0 | 20.4 | 26.0 | 31.4 |
| | CMC | | ∼700 | 2000E | | | |
| C₁₂ | HLB | 1.3 | 8.6 | 14.1 | 19.5 | 25.0 | 30.4 |
| | CMC | ∼6.0 | ∼70 | ∼200 | 225E | | ∼250 |
| C₁₄ | HLB | 0.4 | 7.6 | 13.2 | 18.6 | 24.0 | 29.4 |
| | CMC | | ∼6 | ∼20 | 25-60E | | |
| C₁₆ | HLB | 0.0 | 6.7 | 12.2 | 17.6 | 23.1 | 28.5 |
| | CMC | ∼0.3 | ∼0.6 | ∼4 | | | |
| C₁₈ | HLB | 0.0 | 5.8 | 11.2 | 16.6 | 22.2 | 27.6 |
| | CMC | | | ∼1 | | | |
| E=estimated | | | | | | | |

| | | | | | | | |
|---|---|---|---|---|---|---|---|
| *HLB determined according to Davies: Proc. & International Congress, Surface Activity 1,426, Butterworths, London, 1957. | | | | | | | |
| **ppm | | | | | | | |

As can be seen above, (1) longer pure glucoside chain lengths raise the HLB and lower the molecule's surface activity (high CMC) and (2) the shorter alkyl chain lengths have extremely high CMC's even as the monoglucoside.

### Example XII

| The following formulas were prepared: | | | |
|---|---|---|---|
| | A | B | C |
| Magnesium linear C_{11.2} alkylbenzene sulfonate | 22.4 | 22.4 | 22.4 |
| C₁₂₋₁₃, alkypolyglucoside (G_{1.7}) (<2% free fatty alcohol) | 14.9 | 14.9 | 14.9 |
| C₉₋₁₁ alkoxypropyldihydroxyethyl amine oxide | - | 4 | - |
| C₁₂ alkyldihydroxy ethyl amine oxide | - | - | 4 |
| Ethanol | 5 | 5 | 5 |
| Water | balance | balance | balance |

Formulas A, B and C were compared by generating suds with a constant source of agitation under standard conditions (1 gal water, 115 F. (46.1 C) 0.12 g. hardness in a 3 gal. (11 3 l) dishpan using a standardized mixture of fat plus protein, carbohydrate and edible acid). Dinner plates are washed with 4 ml. of soil on each plate and the suds height is measured after each five plates. 30 plates in total are washed and the integral of the suds height taken over the number of plates washed is reported as the SDW grade (SDW=Suds During Washing).

| | A | B | C |
|---|---|---|---|
| SDW grade | 24 | 28.8 | 28.4 |

This shows that the addition of a small amount of these amine oxides dramatically increases the amount of dishes that can be washed. Similar results are obtained when a fatty acid amide, e.g., a coconut fatty acid amide, diethanol amide, and/or isopropanol amide is substituted, at least in part for the specific amine oxides.

### Example XIII

105 greams of sodium dodecylbenzene sulfonate are mixed with 350 grams of anhydrous sodium sulfate. After the mixture is ground into a fine powder, 70 g of C₁₂₋₁₃ alkylpolyglucoside (G₂₂) (<2% free fatty alcohol) are then mixed in. The mixture is transferred into a fluid bed dryer operated at room temperature (e.g., Aeromatic Inc., Model STREA-1), then 100 grams of a 50% solution of said alkylpolygucoside is sprayed onto the powder, 7.5 milliliters of a 1% polar blue solution are sprayed onto the powder and a small portion of perfume is then added. The resulting granule is dried in a vacuum oven at 30 in. (762 mm) of Hg vacuum at 50°C for ten hours to remove excess water.

### Example XIV

Ammonium C_{11.2} linear ally benzene sulfonate was admixed with C₁₂ alkyLpolyglucoside G_{3.5} in a ratio of about 2:1. The mixture was used at a level of 400 ppm in city water. The initial suds volume was more than 300 ml., but after the addition of about 1.25 grams of a standard grease soil per 200 ml. of wash solution, the suds had disappeared. Substitution of a sodium C₁₂₋₁₆ alkyl glyceryl ether sulfonate for 25% and 40% of the mixture extended the point at which there was no suds to 1.5 and 1.75 grams of soil per 200 ml, of wash solution respectively.

Similar results are obtained when a sodium, potassium, ammonium, or monoethanolammonium C₁₂₋₁₆ alkylpolyethoxy₃ acetates or C₁₄₋₁₆, olefin sulfonate or mixtures thereof is substituted for at least part of the alkyl glyceryl ether sulfonate.

### Example XV

The following formula was prepared with alkylpolyglucosides having 0.3% and 1% free fatty alcohol respectively.

| | Wt. % |
|---|---|
| Ammonium C_{11.2} linear alkyl benzene sulfonate | 17.5 |
| Magnesium C_{11.2} linear alkyl benzene sulfonate | 6.4 |
| Ammonium C₁₂₋₁₃ alkyl polyethoxylate (0.8) sulfate | 6.1 |
| C₁₂-₁₆ alkylpolyglucoside G_{1.7} | 5 |
| Minors and water | balance |

The SDW values for the low (0.3%) and high (1%) free alcohol samples were 12.9 and 12.2 respectively with an LSD_{0.05} at 0.6. See Ex. XII for test method.

### Example XVI

The following formulas were prepared:

| | % by weight | | | |
|---|---|---|---|---|
| | A | B | C | D |
| Ammonium/magnesium C_{11.2} linear alkyl benzene sulfonate | 24.2 | 21.8 | - | - |
| Ammonium/magnesium C₁₂₋₁₅ olefin sulfonate | - | - | 12.8 | 10.6 |
| Ammonium/magnesium C₁₂₋₁₃ alkyl sulfonate | - | - | 19.2 | 15.9 |
| Ammonium C₁₂₋₁₃ alkyl polyethoxylate (0.8) sulfate | 6.5 | 5.8 | - | - |
| C₁₂ fatty acid diethanolamide | - | 3.8 | - | 5.5 |
| C₁₂₋₁₃ alkylpolyglucoside G_{1.7} (free fatty alcohol <0.5%) | 5.3 | 4.8 | 4 | 3.3 |
| Minors and water | balance | | | |
| The SDW Index | A | B | C | D |
| | 79 | 89 | 97 | 107 |

The SDW index is the SDW grade for each product as a percentage of the SDW value of a standard commercial product.

The following are examples of particularly preferred compositions for use in the invention. The broad and preferred ranges of ingredients which can be used are given in the second and third columns, respectively, in each example.

### Example XVII

| | % by weight | | |
|---|---|---|---|
| Ammonium C_{11.4} alkyl benzene sulfonate | 17.5 | 10-35 | 12-25 |
| Magnesium C_{11.4} alkyl benzene sulfonate | 6.4 | 0-11 | 3-9 |
| Ammonium C₁₂₋₁₃ alkyl polyethoxylate (0.8) sulfates | 6.1 | 2-11 | 3-9 |
| C₁₂₋₁₃ alkyl polyglucoside (1.7) derived from glucose (<0.5% free fatty alcohol) | 5.0 | 2-11 | 2-7 |
| Ethanol | 3.7 | 0-10 | 0-5 |
| Ammonium xylene sulfonate | 3.0 | 0-10 | 0-5 |
| H₂O & minor components, e.g., perfume | Balance | | |

### Example XVIII

| | % by weight | | |
|---|---|---|---|
| Ammonium C₁₂₋₁₃ alkyl sulfate | 15.7 | 7-23 | 10-20 |
| Sodium C₁₄₋₁₆ olefin sulfonate | 10.4 | 4-19 | 6-13 |
| MgCl₂ · 6H₂O | 5.6 | 0-11 | 2-10 |
| Coconut monoethanol amide | 5.5 | 2-8 | 3-7 |
| C₁₂₋₁₃ alkyl polyglycoside (1.7) derived from glucose (<0.5% free fatty alcohol) | 5.9 | 2-12 | 3-9 |
| Ethanol | 4.0 | 0-10 | 0-10 |
| H₂O and minor components, e.g., perfume | Balance | | |

The alkyl groups in the surfactants of Examples XVII and XVIII can vary from about 10 to about 16 carbon atoms and the cations can be ammonium, sodium, potassium, monoethanolammonium, diethanolammonium, triethanolammonium, magnesium, or preferably, mixtures thereof. Any of the preferred alkyl polyglycosides can be used and other known amine oxide and amide suds boosters disclosed herein can be used.

### Example XIX

When a 2:1 mixture of an ammonium C_{11.2}, alkylbenzene sulfonate and the C₁₂₋₁₃ alkylpolyglucoside (2-4) (<2% free fatty alcohol) are tested under the conditions of Example II the initial suds volume is good, but the SDW grade is not as good as some premium commercial products. Substitution of between 25% and 50% of the mixture with a sodium C₁₂₋₁₆ alkyl glyceryl ether sulfonate, or sodium C₁₄₋₁₆ olefin sulfonate, or sodium C₁₂₋₁₃ alkyl polyethoxylate₍₃₎ acetate increases the SDW grade without lowering the initial sudsing excessively.

Known analytical techniques can be used to determine the structures of the alkylpolysaccharide surfactants herein; for example, to determine the glycosidic chain length, the amount of butyl glucoside, the free fatty alcohol content, and the level of unreacted polysaccharide. More specifically, gas or liquid chromatography can be used to determine the unreacted alcohol content and the unreacted polysaccharide content respectively. Proton nmr can be used to determine the average glycosidic chain length. The point of attachment of the hydrophilic portion of the molecule to the hydrophobic portion of the molecule can be determined by ¹³C nmr.

The alkylpolyglucoside surfactants are complex mixtures. Their components vary depending upon the nature of the starting materials and the reaction by which they are prepared. Analytical standards which are useful in calibrating instruments for analyzing the components of a particular alkylpolyglucoside surfactant can be obtained from Calbiochem Behring Co. LaJolla, California. These standards include those for octylglucoside (Calbiochem #494559), decylglucoside (Calbiochem #252715), dodecylmaltoside (Calbiochem #3243555).

The HLBs of alkylpolyglucoside surfactants useful in the foaming compositions for use in the invention have the values given in Example XV the CMCs will approximate those values given in the same example. Alkylpolyglucoside surfactants having the structures specified in the claims and characterized by one or more of the standard analytical techniques will give the results indicated in the examples.

## Claims

1. Use as a foaming dishwashing composition of a composition comprising:
an alkylpolyglucoside surfactant having the formula
RO(R¹O)ₜZₓ
wherein Z is a moiety derived from glucose, R is an alkyl group containing 12 to 18 carbon atoms, R¹ is ethylene, propylene and/or glyceryl, t is from 0 to 10, and x is a number from 1.5 to 4;
(2) an anionic cosurfactant selected from the group consisting of sulfates, sulfonates, carboxylates and mixtures thereof, neutralised with one or more cationic moieties, the weight ratio of (2) to (1) being from 1:10 to 10:1 and wherein the alkylpolyglucoside contains less than 50% short chain alkyl polyglucoside and less than 10% unreacted fatty alcohol except that:
(1) the weight ratio of (2) to (1) is superior to 1:1 when the anionic surfactant is an alkalimetal alkylbenzensulfonate and when in the general formula for the alkylpolyglucoside t=0;
(2) the weight ratio of (2) to (1) is at least 1:2 when the anionic cosurfactant is soap; and
(3) when the anionic cosurfactant does not contain a sulfonate or carboxylate x must be from 1.5 to and the alkylpolyglucoside surfactant must have a free fatty alcohol content of less than 2% by weight.

2. The use of claim 1 wherein the cosurfactant is selected from the group consisting of alkylbenzene sulfonates, alpha-olefin sulfonates, alkyl sulfates and paraffin sulfonates and wherein the cationic moiety is selected from the group consisting of sodium, potassium, ammonium, monoethanolammonium, diethanolammonium, triethanolammonium, calcium, magnesium and mixtures thereof.

3. The use of claim 2 wherein the cosurfactant is an alkylbenzene sulfonate.

4. The use of claim 3 wherein the phenyl portion of the alkylbenzene sulfonate is attached near the middle of the alkyl chain and the cationic moiety is magnesium.

5. The use of claim 2 wherein the cosurfactant is an alpha-olefin sulfonate.

6. The use of claim 2 wherein the cosurfactant is a paraffin sulfonate.

7. The use of claim 1 wherein the anionic cosurfactant has the formula
R⁹(SO₃)_{y}(COO)_{z}M_{q}
wherein R⁹ is an alkyl, alkylphenyl, hydroxyalkylphenyl or hydroxylalkyl or mixtures thereof, said alkyl groups containing from 6 to 30 carbon atoms; wherein y is a number from 0 to 4, z is a number from 0 to 4, y+z is at least 1 and wherein M is a cationic moiety with q being selected to complete the formula, x is from 1.5 to 3 and the alkyl polyglucoside surfactant has a free fatty alcohol content of less than 2% by weight.

8. The use of claim 7 wherein y is 0; z is 1; and wherein the cationic moiety is selected from the group consisting of sodium, potassium, ammonium, monoethanolammonium, diethanolammonium, triethanolammoniun, calcium, magnesium and mixtures thereof.

9. The use as a foaming dishwashing composition of a light-duty liquid detergent composition comprising from 5% to 50% by weight of the surfactant mixture defined in claim 1 and from 1% to 50% by weight of a solvent selected from the group consisting of C₁₋₃, alkanols, C₁₋₃ alkanolamines, C₂₋₄ polyols, and mixtures thereof, and the balance water.

10. The use of claim 9 wherein the cosurfactant is selected from the group consisting of alkylbenzene sulfonates, alpha-olefin sulfonates, alkyl sulfates and paraffin sulfonates and the cationic moiety is selected from the group consisting of sodium, potassium, ammonium, monoethanolammonium, diethanolammonium, triethanolammonium, calcium, magnesium and mixtures thereof.

11. The use of claim 10 wherein the cosurfactant is an alkylbenzene sulfonate.

12. The use of claim 10 wherein the cationic moiety is magnesium.

13. The use of claim 10 wherein the cosurfactant is an alpha-olefin sulfonate.

14. The use of claim 10 wherein the cosurfactant is a paraffin sulfonate.

## Patentansprüche

1. Verwendung als schäumende Geschirrspülmittelzusammensetzung einer Zusammensetzung, umfassend:
(1) ein Alkylpolyglucosid-Tensid der Formel
RO(R¹O)ₜZₓ
worin Z eine von Glucose abgeleitete Einheit ist, R eine 12 bis 18 Kohlenstoffatome enthaltene Alkylgruppe ist, R¹ Ethylen, Propylen und/oder Glyceryl ist, t 0 bis 10 ist, und x eine Zahl von 1,5 bis 4 ist:
(2) ein anionisches Cotensid, gewählt aus der Sulfate, Sulfonate, Carboxylate und Mischungen hiervon umfassenden Gruppe, welches mit einer oder mehreren kationischen Gruppen neutralisiert ist, wobei das Gewichtsverhältnis von (2) zu (1) 1:10 bis 10:1 beträgt und wobei das Alkylpolyglucosid weniger als 50% kurzkettiges Alkylpolyglucosid und weniger als 10% nicht-reagierten Fettalkohol enthält, mit der Ausnahme, daß
(1) das Gewichtsverhältnis von (2) zu (1) über 1:1 liegt, wenn das anionische Tensid ein Alkalimetallalkylbenzolsulfonat ist und wenn in der allgemeinen Formel für das Alkylpolyglucosid t=0:
(2) das Gewichtsverhältnis von (2) zu (1) mindestens 1:2 beträgt, wenn das anionische Cotensid Seife ist; und
(3) wenn das anionische Cotensid kein Sulfonat oder Carboxylat enthält, x 1,5 bis 3 betragen muß, und das Alkylpolyglucosid-Tensid einen Gehalt an freiem Fettalkohol von weniger als 2 Gew.-% aufweisen muß.

2. Verwendung nach Anspruch 1, wobei das Cotensid aus der Alkylbenzolsulfonate, alpha-Olefinsulfonate, Alkylsulfate und Paraffinsulfonate umfassenden Gruppe gewählt ist, und wobei die kationische Gruppe aus der Natrium, Kalium, Ammonium, Monoethanolammonium, Diethanolammonium, Triethanolammonium, Calcium, Magnesium und Mischungen hiervon umfassenden Gruppe gewählt ist.

3. Verwendung nach Anspruch 2, wobei das Cotensid ein Alkylbenzolsulfonat ist.

4. Verwendung nach Anspruch 3, wobei der Phenylteil des Alkylbenzolsulfonats nahe der Mitte der Alkylkette gebunden ist und die kationische Gruppe Magnesium ist.

5. Verwendung nach Anspruch 2, wobei das Cotensid ein alpha-Olefinsulfonat ist.

6. Verwendung nach Anspruch 2, wobei das Cotensid ein Paraffinsulfonat ist.

7. Verwendung nach Anspruch 1, wobei das anionische Cotensid der Formel
R⁹(SO₃)_{y}(COO)_{z}M_{q}
entspricht, worin R⁹ ein Alkyl, Alkylphenyl, Hydroxyalkylphenyl oder Hydroxyalkyl oder Mischungen hiervon bedeutet, wobei die Alkylgruppen 6 bis 30 Kohlenstoffatome enthalten; y eine Zahl von 0 bis 4 ist; z eine Zahl von 0 bis 4 ist, y+z mindestens 1 ist und wobei M eine kationische Gruppe ist, wobei q so gewählt ist, um die Formel zu vervollständigen, x 1,5 bis 3 ist und das Alkylpolyglucosid-Tensid einen Gehalt an freiem Fettalkohol von weniger als 2 Gew.-% aufweist.

8. Verwendung nach Anspruch 7, wobei y 0 ist; z 1 ist; und wobei die kationische Gruppe aus der Natrium, Kalium, Ammonium, Monoethanolammonium, Diethanolammonium, Triethanolammonium, Calcium, Magnesium und Mischungen hiervon umfassenden Gruppe gewählt ist.

9. Verwendung als schäumende Geschirrspülmittelzusammensetzung einer flüssigen Feinwaschmittelzusammensetzung, umfassend 5 bis 50 Gew.-% der in Anspruch 1 definierten Tensidmischung und 1 bis 50 Gew. -% eines aus der C₁₋₃-Alkanole, C₁₋₃-Alkanolamine, C₂₋₄-Polyole und Mischungen hiervon umfassenden Gruppe gewählten Lösungsmittels, und als Rest Wasser.

10. Verwendung nach Anspruch 9, wobei das Cotensid aus der Alkylbenzolsulfonate, alpha-Olefinsufonate, Alkylsulfate und Paraffinsulfate umfassenden Gruppe gewählt ist, und die kationische Gruppe aus der Natrium, Kalium, Ammonium, Monoethanolammonium, Diethanolammonium, Triethanolammonium, Calcium, Magnesium und Mischungen hiervon umfassenden Gruppe gewählt ist.

11. Verwendung nach Anspruch 10, wobei das Cotensid ein Alkylbenzolsulfonat ist.

12. Verwendung nach Anspruch 10, wobei die kationische Gruppe Magnesium ist.

13. Verwendung nach Anspruch 10, wobei das Cotensid ein alpha-Olefinsulfonat ist.

14. Verwendung nach Anspruch 10, wobei das Cotensid ein Paraffinsulfonat ist.

## Revendications

1. Utilisation, comme composition moussante pour le lavage de la vaisselle, d'une composition comprenant:
(1) un tensioactif alkylpolyglucoside de formule
RO(R¹O)ₜZₓ
dans laquelle Z est un fragment dérivé du glucose, R est un groupe alkyle contenant 12 à 18 atomes de carbone, R₁ est un éthylène, un propylène et/ou un glycéryle, t vaut de 0 à 10 et x est un nombre de 1,5 à 4;
(2) un cotensioactif anionique choisi dans le groupe constitué par les sulfates, les sulfonates, les carboxylates et leurs mélanges, neutralisé avec un ou plusieurs fragments cationiques, le rapport pondéral de (2) à (1) étant de 1:10 à 10:1, et dans lequel l'alkylpolyglucoside contient moins de 50% d'alkylpolyglucoside à chaîne courte et moins de 10% d'alcool gras n'ayant pas réagi, sauf que:
(1) le rapport pondéral de (2) à (1) est supérieur à 1:1 lorsque le tensioactif anionique est un alkylbenzènesulfonate de métal alcalin et lorsque, dans la formule générale de l'alkylpolyglycoside, t=0;
(2) le rapport pondéral de (2) à (1) est d'au moins 1:2 lorsque le cotensioactif anionique est un savon; et
(3) lorsque le cotensioactif anionique ne contient ni sulfonate ni carboxylate, x doit avoir de 1,5 à 3 et le tensioactif alkylpolyglucoside doit avoir une teneur en alcool gras libre inférieure à 2% en poids.

2. Utilisation selon la revendication 1, dans laquelle le cotensioactif est choisi dans le groupe comprenant les alkylbenzènesulfonates, les alphaoléfinesulfonates, les alkylsulfates et les paraffinesulfonates, et dans laquelle le fragment cationique est choisi dans le groupe comprenant le sodium, le potassium, l'ammonium, le monoéthanolammonium, le diéthanolammonium, le triéthanolammonium, le calcium, le magnésium et leurs mélanges.

3. Utilisation selon la revendication 2, dans laquelle le cotensioactif est un alkylbenzènesulfonate.

4. Utilisation selon la revendication 3, dans laquelle le fragment phényle de l'alkylbenzènesulfonate est fixé au voisinage du milieu de la chaîne alkyle, le fragment cationique étant le magnésium.

5. Utilisation selon la revendication 2, dans laquelle le cotensioactif est un alpha-oléfinesulfonate.

6. Utilisation selon la revendication 2, dans laquelle le cotensioactif est un paraffinesulfonate.

7. Utilisation selon la revendication 1, dans laquelle le cotensioactif anionique a la formule
R⁹(SO₃)_{y}(COO)_{z}M_{q}
dans laquelle R⁹ est un radical alkyle, alkylphényle, hydroxyalkylphényle ou hydroxyalkyle ou leurs mélanges, lesdits groupes alkyle contenant de 6 à 30 atomes de carbone; dans laquelle y est un nombre de 0 à 4, z est un nombre de 0 à 4, y + z vaut au moins 1, et dans laquelle M est un fragment cationique, q étant choisi de façon à compléter la formule, x vaut de 1,5 à 3 et le tensioactif alkylpolyglucoside présente une teneur en alcool gras libre inférieure à 2% en poids.

8. Utilisation selon la revendication 7, dans laquelle y vaut 0; z vaut 1; et dans laquelle le fragment cationique est choisi dans le groupe comprenant le sodium, le potassium, l'ammonium, le monoéthanolammonium, le diéthanolammonium, le triéthanolammonium, le calcium, le magnésium et leurs mélanges.

9. Utilisation, comme composition moussante pour le lavage de la vaisselle, d'une composition détergente liquide pour lavage délicat, comprenant de 5 à 50% en poids du mélange de tensioactifs défini dans la revendication 1 et de 1 à 50% en poids d'un solvant choisi dans le groupe comprenant les alcanols en C₁₋₃, les alcanolamines en C₁₋₃, les polyols en C₂₋₄ et leurs mélanges, le reste étant de l'eau.

10. Utilisation selon la revendication 9, dans laquelle le cotensioactif est choisi dans le groupe comprenant les alkylbenzènesulfonates, les alpha-oléfinesulfonates, les alkylsulfates et les paraffinesulfonates, le fragment cationique étant choisi dans le groupe comprenant le sodium, le potassium, l'ammonium, le monoéthanolammonium, le diéthanolammonium, le triéthanolammonium, le calcium, le magnésium et leurs mélanges.

11. Utilisation selon la revendication 10, dans laquelle le cotensioactif est un alkylbenzènesulfonate.

12. Utilisation selon la revendication 10, dans laquelle le fragment cationique est le magnésium.

13. Utilisation selon la revendication 10, dans laquelle le cotensioactif est un alpha-oléfinesulfonate.

14. Utilisation selon la revendication 10, dans laquelle le cotensioactif est un paraffinesulfonate.
